# EUROPÄISCHE PATENTSCHRIFT

(11) **EP 1 029 919 B1**
(45) Veröffentlichungstag und Bekanntmachung des Hinweises auf die Patenterteilung: **31.03.2004**
(21) Anmeldenummer: 00102601.2
(22) Anmeldetag: 08.02.2000
(51) Int. Cl.: C12N 15/53, C12N 9/06, C12P 13/06, C12P 13/08, C12P 13/22

(54) **Verfahren zur fermentativen Herstellung von L-Aminosäuren unter Verwendung coryneformer Bakterien**
Process for the fermentative production of L-amino acids using coryneform bacteria
Procédé de production de L-aminoacides par fermentation de bactéries coryneformes

(30) Priorität: 20.02.1999 DE 19907347; 03.06.1999 US 324940
(43) Veröffentlichungstag der Anmeldung: 23.08.2000
(73) Patentinhaber: Degussa AG, 40474 Düsseldorf (DE); Forschungszentrum Jülich GmbH, 52425 Jülich (DE)
(72) Erfinder: Marx, Achim, Dr., 33613 Bielefeld (DE); Möckel, Bettina, Dr., 33602 Bielefeld (DE); Pfefferle, Walter, Dr., 33790 Halle (DE); Sahm, Hermann, Dr., 52428 Jülich (DE); De Graaf, Albert, Dr., 6418 CB Heerlen (NL); Eggeling, Lothar, Dr., 52428 Jülich (DE)

(56) Entgegenhaltungen:
- EP-A- 0 358 940
- EP-A- 0 435 132
- WO-A-99/46363
- FR-A- 2 575 492
- MARX A ET AL.: "Response of the central metabolism in Corynebacterium glutamicum to the use of an NADH-dependent glutamate dehydrogenase." METABOLIC ENGINEERING, Bd. 1, Nr. 1, Januar 1999 (1999-01), Seiten 35-48, XP002154493
- MARX A: "Bestimmung des Kohlenstoffflusses im Zentralstoffwechsel von Corynemacterium glutamicum mittels 13C-isotopenanalyse" BERICHTE DES FORSCHUNGSZENTRUMS JUELICH, ISSU 3459, FORSCHUNGSZENTRUM JUELICH, ZENTRALBIBLIOTHEK, JUELICH, DE, 1997, XP002154494 ISSN: 0944-2952
- KRAMER R: "Genetic and physiological approaches for the production of amino acids" JOURNAL OF BIOTECHNOLOGY, Bd. 45, Nr. 1, 12. Februar 1996 (1996-02-12), Seiten 1-21, XP004036833 ISSN: 0168-1656
- BÖRMANN E R ET AL.: "Molecular analysis of the Corynebacterium glutamicum gdh gene encoding glutamate dehydrogenase" MOLECULAR MICROBIOLOGY, Bd. 6, Nr. 3, 1992, Seiten 317-326, XP000914881 ISSN: 0950-382X
- SNEDECOR B ET AL.: "Selection, expression, and nucleotide sequencing of the glutamate dehydrogenase gene of Streptococcus asaccharolyticus" JOURNAL OF BACTERIOLOGY, Bd. 173, Nr. 9, Oktober 1991 (1991-10), Seiten 6162-6167, XP000914882 ISSN: 0021-9193

## Beschreibung

Gegenstand der Erfindung ist ein Verfahren zur fermentativen Herstellung von L-Aminosäuren unter Verwendung von coryneformen Bakterien, in denen das Glutamatdehydrogenase-Gen insbesondere überexprimiert wird.

### Stand der Technik

L-Aminosäuren finden in der Tierernährung, in der Humanmedizin und in der pharmazeutischen Industrie Anwendung.

L-Aminosäuren werden fermentativ mit L-Aminosäuren produzierenden Stämmen coryneformer Bakterien insbesondere mit Corynebacterium glutamicum hergestellt. Wegen der großen Bedeutung dieser Produktgruppe wird ständig an der Verbesserung des Herstellverfahrens gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikrorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Aminosäuren sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Biosynthesegene amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, Londön, UK, 1985, 115-142), Hilliger (BioTec 2, 40-44 (1991)), Jetten und Sinskey (Critical Reviews in Biotechnology 15, 73-103 (1995)) und Sahm et al. (Annuals of the New York Academy of Science 782, 25-39 (1996))

Das Enzym Glutamat-Dehydrogenase katalysiert die reduktive Aminierung von α-Ketoglutarsäure zu Glutaminsäure. In der französischen Offenlegungsschrift 2 575 492 wird ein DNA-Fragment aus Corynebacterium melassecola 801 beschrieben, das ein Glutamat-Dehydrogenase-Gen trägt. Möglicherweise setzt man es dort zur Erhöhung der Glutaminsäurebildung bei der Fermentation von Corynebacterium melassecola ein, um ausgehend von Glutamat die Aminosäuren Glutamin, Prolin und Arginin herzustellen. Die Nukleotidsequenz des Glutamat-Dehydrogenase-Gens von Corynebacterium glutamicum ATCC13032 wurde von'Börmann et al. (Molekular Microbiology 6, 317-326 (1992)) beschrieben. Das Zitat enthält nur Angaben über die Erhöhung der spezifischen Aktivität des Enzyms in der Zelle.

Aus der WO 99/46363 ist die Herstellung von L-Lysin mit Corynebacterium bekannt, das mit einem Peptostreptococcus asaccharolyticus stammenden gdh-Gen transformiert wurde.

Die Nukleotidsequenz des Glutamat-Dehydrogenase-Gens von Peptostreptococcus asaccharolyticus findet sich bei Snedecor et al. (Journal of Bacteriology 173, 6162-6167 (1991))

### Aufgabe der Erfindung

Die Erfinder haben sich zur Aufgabe gestellt, neue Maßnahmen zur verbesserten fermentativen Herstellung von anderen L-Aminosäuren bereitzustellen.

### Beschreibung der Erfindung

L-Aminosäuren finden in der Tierernährung, in der Humanmedizin und in der pharmazeutischen Industrie Anwendung. Es besteht daher ein allgemeines Interesse daran neue, verbesserte Verfahren zur Herstellung von L-Aminosäuren bereitzustellen.

Wenn im folgenden L-Aminosäuren erwähnt werden, sind damit die Protein-bildenden Aminosäuren L-Threonin, L-Isoleucin, L-Valin, L-Tryptophan und gegebenenfalls deren Salze gemeint, insbesondere L-Threonin und L-Tryptophan.

Gegenstand der Erfindung ist ein Verfahren zur Herstellung von einer oder mehreren der L-Aminosäuren, ausgewählt aus der Gruppe der L-Threonin, L-Isoleucin, L-Valin und L-Tryptophan durch Fermentation coryneformer Bakterien, dadurch gekennzeichnet, daß man mit einem aus Mikroorganismen stammenden transformierte Bakterien einsetzt, in denen man die intrazelluläre Aktivität der Glutamatdehydrogenase erhöht, insbesondere die für das Glutamatdehydrogenase-Gen codierende Nukleotidsequenz überexprimiert.

Bevorzugte Ausführungsformen finden sich in den Ansprüchen.

Die Erhöhung der intrazellulären Aktivität eines oder mehrerer Enzyme in einem Mikroorganismus, die durch die entsprechende DNA kodiert werden, wird dadurch bewirkt, dass indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte Mutanten bzw. Stämme, wie beispielsweise
die L-Threonin produzierenden Stämme
Corynebacterium glutamicum FERM-P 5835
Brevibacterium flavum FERM-P 4164 und
Brevibacterium lactofermentum FERM-P 4180,
oder die L-Isoleucin produzierenden Stämme
Corynebacterium glutamicum FERM-P 756
Brevibacterium flavum FERM-P 759 und
Brevibacterium lactofermentum FERM-P 4192
oder die L-Valin produzierenden Stämme
Brevibacterium flavum FERM-P 512 und
Brevibacterium lactofermentum FERM-P 1845,
und die L-Tryptophan produzierenden Stämme
Corynebacterium glutamicum FERM-BP 478
Brevibacterium flavum FERM-BP 475 und
Brevibacterium lactofermentum FERM-P 7127

Die Erfinder fanden heraus, daß coryneforme Bakterien nach Überexpression der L-Glutamatdehydrogenase in verbesserter Weise L-Aminosäuren produzieren, wobei die L-Glutaminsäure hier nicht beansprucht wird.

Das von Börmann et al. (Molecular Microbiology 6, 317-326 (1992)) beschriebene Glutamat-Dehydrogenase Gen von C. glutamicum kann erfindungsgemäß verwendet werden. Weiterhin eignet sich das Glutamat-Dehydrogenase Gen aus anderen Mikroorganismen wie z. B. das aus Peptostreptococcus asaccharolyticus, das vow Snedecor et al. (Journal of Bacteriology 173, 6162-6167 (1991)) beschrieben wurde. Weiterhin können Allele der genannten Gene verwendet werden, die sich aus der Degeneriertheit des genetischen Codes oder durch funktionsneutrale Sinnmutationen (sense mutations) ergeben.

Zur Erzielung einer Überexpression kann die Kopienzahl der entsprechenden Gene erhöht werden, oder es kann die Promotor- und Regulationsregion, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin. eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispiele für Plasmide mit Hilfe derer die Glutamat-Dehydrogenase überexprimiert werden kann sind pEK1,9gdh-1 und pEKExpgdh, die in den Stämmen ATCC13032/pEK1,9gdh-1 und DH5α/pEKExpgdh enthalten sind. Plasmid pEK1,9gdh-1 ist ein Pendelvektor, der die NADP abhängige Glutamat-Dehydrogenase Gen von C. glutamicum enthält. Plasmid pEKExpgdh ist ein Pendelvektor, der die NAD abhängige Glutamat-Dehydrogenase von Peptostreptococcus asaccharolyticus enthält.

Zusätzlich kann es für die Produktion der entsprechenden L-Aminosäure vorteilhaft sein neben der Glutamat-Dehydrogenase ein oder mehrere Enzyme des jeweiligen Aminosäure-Biosyntheseweges zu überexprimieren. So kann beispielsweise
- zur Verbesserung von L-Valin produzierenden coryneformen Bakterien zusätzlich das für die Acetohydroxysäure-Synthase kodierenden Gen überexprimiert werden (EP-B 0356739),
- zur Verbesserung von L-Tryptophan produzierenden coryneformen Bakterien zusätzlich das für die Anthranilsäurephosphoribosyl Transferase kodierende Gen überexprimiert werden (EP-B 0124048),
- zur Verbesserung von L-Threonin oder L-Isoleucin produzierenden coryneformen Bakterien zusätzlich das für die Homoserin-Dehydrogenase kodierende Gen überexprimiert werden (EP-A 0131171).

Weiterhin kann es für die Produktion der entsprechenden L-Aminosäure vorteilhaft sein neben der Überexpression der Glutamat-Dehydrogenase unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (.Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum der gewünschten L-Aminosäure gebildet hat. Dieses Ziel wird normalerweise innerhalb von 10 Stunden bis 160 Stunden erreicht.

Die Analyse von L-Aminosäuren kann automatisch, basierend auf einer Anionenaustauschchromatographie mit anschließender Ninhydrin Derivatisierung erfolgen so wie bei Spackman et al. (Analytical Chemistry, 30, 1190 (1958)) beschrieben.

Folgende Mikroorganismen wurden bei der Deutschen Sammlung für Mikrorganismen und Zellkulturen (DSMZ, Braunschweig, Deutschland) gemäß Budapester Vertrag hinterlegt:
Corynebacterium glutamicum Stamm ATCC13032/pEK1,9gdh-1 als DSM 12614.
Escherichia coli K12 Stamm DH5α/pEKExpgdh als DSM 12613.

### Beispiele

Die vorliegende Erfindung wird im folgenden anhand von Ausführungsbeispielen näher erläutert.

Zu diesem Zweck wurden Versuche mit Aminosäuren produzierenden Stämmen durchgeführt, in denen die Überlegenheit des beanspruchten Verfahrens demonstriert wird:
a) der L-Threonin und L-Isoleucin produzierende Stamm Brevibacterium flavum DSM5399 (EP-B- 0385 940) und
b) der L-Valin produzierende, Isoleucin-bedürftige Stamm ATCC13032ΔilvA, der als DSM12455 bei der Deutschen Sammlung für Mikroorganismen und Zellkulturen in Braunschweig (Deutschland) gemäss Budapester Vertrag hinterlegt worden ist.

### Beispiel 1

### Herstellung von L-Aminosäure-Produzenten mit verstärkter Glutamat-Dehydrogenase

Das Plasmid pEK1,9gdh-1 entspricht dem von Börmann et al. (Molecular Microbiology 6, 317-326 (1992)) beschriebenem Plasmid pEK1.9gdh. Es wurde aus ATCC.13032/pEK1,9gdh-1 isoliert. In gleicher Weise wurde das bekannte Plasmid pEKExpgdh (Marx et al., Metabolic Engineering 1, 35-48 (1999)), welches das Glutamat-Dehydrogenase Gen von Peptostreptococcus asaccharolyticus (Snedecor et al., Journal of Bacteriology 173, 6162-6167 (1991)) trägt, aus dem E. coli Stamm DH5α/pEKExpgdh isoliert.

Die Stämme DSM5399 und ATCC13032ΔilvA wurden wie bei Liebl et al. (FEMS Microbiology Letters 65, 299-304 (1989)) mit dem Plasmid pEK1,9gdh-1 transformiert. Die Selektion der Transformanten erfolgte auf Hirn-Herz-Agar der Firma Merck (Darmstadt, Deutschland), der mit 50 mg/l Kanamycin supplementiert worden war. Auf diese Weise entstanden die Stämme DSM5715/pEK1,9gdh-1, DSM5399/pEK1,9gdh-1 und ATCC13032ΔilvA/pEK1,9gdh-1. In gleicher Weise wurde der Stamm DSM5715 mit dem Plasmid pEKExpgdh transformiert und der Stamm DSM5715/pEKExpgdh erhalten.

**Tabelle 1**

| Komponente | Konzentration Pro Liter |
|---|---|
| (NH₄)₂SO₄ | 20 g |
| Harnstoff | 5 g |
| KH₂PO₄ | 1 g |
| K₂HPO₄ | 1 g |
| MgSO₄ · 7H₂O | 0,25 g |
| 3-Morpholinopropansulfonsäure | 42 g |
| FeSO₄ · 7H₂O | 10 mg |
| MnSO₄ · H₂O | 10 mg |
| ZnSO₄ · 7H₂O | 1 mg |
| CuSO₄ | 0,2 mg |
| NiCl₂ · 6H₂O | 0, 02 mg |
| CaCl₂ | 10 mg |
| Protocatecholsäure | 0,03 mg |
| Biotin | 200 pg |
| Medium CgXII (Keilhauer et al.) | |

Nach der Fermentation wurden die optische Dichte (OD) (Biochrom Novaspec 4049, LKB Instrument GmbH, Gräfelfing, Deutschland) bei einer Messwellenlänge von 600 nm und die Konzentration an gebildeter L-Aminosäure mit einem Aminosäureanalysator der Firma Eppendorf-BioTronik (Hamburg, Deutschland) durch Ionenaustauschchromatographie und Nachsäulenreaktion mit Ninhydrindetektion bestimmt. In den Tabellen 2 und 3 sind die das Ergebnisse der Versuche dargestellt.

### Beispiel 2

### Herstellung von L-Threonin und L-Isoleucin

Stamm DSM5399/pEK1,9gdh-1 wurde in Komplettmedium CgIII(Kase & Nakayama, Agricultural and Biological Chemistry 36 (9) 1611- 1621 (1972)) mit 50µg/ml Kanamycin vorkultiviert. Hierzu wurde 10 ml Medium CgIII, die in 100 ml Erlenmeyerkolben mit 4 Schikanen enthalten waren, mit einer Impföse des Stammes angeimpft und die Kultur für 16 Stunden bei 240 rpm und 30°C bebrütet.

Zur Inokulierung von 10 ml Produktionsmedium, das in 100ml Erlenmeyerkolben mit 4 Schikanen enthalten war, wurde die OD (660nm) der Vorkultur bestimmt. Die Hauptkultur wurde auf eine OD von 0,1 angeimpft. Als Produktionsmedium wurde das von Keilhauer et al., beschriebene Medium CgXII verwendet (Journal of Bacteriology 1993, 175: 5595 - 5603). Die Zusammensetzung des Mediums ist in Tabelle 1 dargestellt. Es wurden 4% Glukose und 50mg/l Kanamycinsulfat zugesetzt. Inkubiert wurden die Zellen bei 33°C, 250 rpm und 80% Luftfeuchte für 48 Stunden. Anschließend wurde die optische Dichte bei 660nm und die Konzentration an gebildetem L-Threonin und L-Isoleucin mit wie oben angegeben bestimmt. In Tabelle 2 ist das Ergebnis des Versuches dargestellt.

**Tabelle 2**

| Stamm | OD | L-Threonin g/l | L-Isoleucin g/l |
|---|---|---|---|
| DSM5399 | 10.5 | 1,77 | 1,05 |
| DSM5399/pEK1,9gdh-1 | 11.0 | 2,26 | 1,44 |

### Beispiel 3

### Herstellung von L-Valin

Stamm ATCC13032ΔilvA/pEK1,9gdh-1 wurde in Komplettmedium CgIII (Kase & Nakayama, Agricultural and Biological Chemistry 36 (9) 1611- 1621 (1972)) mit 50µg/ml Kanamycin vorkultiviert. Hierzu wurde 50 ml Medium CgIII, die in 500 ml Erlenmeyerkolben mit 4 Schikanen enthalten waren, mit einer Impföse des Stammes angeimpft und die Kultur für 16 Stunden bei 140 rpm und 30°C bebrütet.

Zur Inokulierung von 60 ml Produktionsmedium, das in 500ml Erlenmeyerkolben mit 4 Schikanen enthalten war, wurde die OD (660nm) der vorkultur bestimmt. Die Hauptkultur wurde abzentrifugiert und der Überstand verworfen. Das Pellet wurde in 5 ml Produktionsmedium aufgenommen und die Hauptkultur auf eine OD von 0,3 angeimpft. Als Produktionsmedium wurde Medium CgXII (Keilhauer et al., Journal of Bacteriology 1993 175: 5595 - 5603) wie in Beispiel 2 beschrieben (mit 4% Glucose) verwendet. Inkubiert wurden die Zellen bei 30°C, 150 rpm für 48h.

Anschließend wurde die optische Dichte bei 660nm und die Konzentration an gebildetem L-Valin wie oben angegeben bestimmt. In Tabelle 3 ist das Ergebnis des Versuches dargestellt.

**Tabelle 3**

| Stamm | OD | L-Valin g/l |
|---|---|---|
| ATCC13032ΔilvA | 18,5 | 0,29 |
| ATCC13032ΔilvA /pEK1,9gdh-1 | 17,6 | 0,45 |

### Beispiel 4

### Herstellung von L-Lysin, L-Valin und L-Alanin

Stamm DSM5715/pEKExpgdh wurde in Komplexmedium 2TY bestehend aus 16 g/l Trypton, 10 g/l Hefeextrakt und 5 g/l NaCl vorkultiviert. Hierzu wurden 60 ml Medium 2TY, die in 500 ml Erlenmeyerkolben mit 2 Schikanen enthalten waren, mit einer Impföse des Stammes angeimpft und die Kultur für 12 Stunden bei 150 rpm und 30°C bebrütet.

Zur Inokulierung von 60 ml Produktionsmedium, das in 500 ml Erlenmeyerkolben mit 2 Schikanen enthalten war, wurde die Vorkultur bei 5000 rpm in einer Sepatech Minifuge RF (Heraeus, Hanau, Deutschland) Zentrifuge für 10 Minuten abzentrifugiert. Der Überstand wurde verworfen und das Pellet wurde in 1 ml Produktionsmedium resuspendiert. Ein Aliquot dieser Zellsuspension wurde dem Produktionsmedium zugesetzt, so daß eine OD600 von ca. 0,4 vorlag. Als Produktionsmedium wurde das von Schrumpf et al. (Journal of Bacteriology 173, 4510-4516 (1991)) beschriebene Medium CGC (Tabelle 5) supplementiert mit 25 g/l Glucose, 350 mg/l Leucin, 42 g/l 3-Morpholinopropansulfonsäure und 50 mg/l Kanamycinmonosulfat bei pH 7 verwendet. Die Kulturen wurden für 30 Stunden bei 30°C und 150 rpm bebrütet.

**Tabelle 4**

| Komponente | Konzentration Pro Liter |
|---|---|
| (NH₄)₂SO₄ | 5 g |
| Harnstoff | 5 g |
| KH₂PO₄ | 0,5 g |
| K₂HPO₄ | 0,5 g |
| MgSO₄ · 7H₂O | 0,25 g |
| FeSO₄ · 7H₂O | 10 mg |
| MnSO₄ · H₂O | 10 mg |
| ZnSO₄ · 7H₂O | 1 mg |
| CuSO₄ | 0, 2 mg |
| NiCl₂ · 6H₂O | 0,02 mg |
| CaCl₂ · 2H₂O | 10 mg |
| Biotin | 200 µg |

Anschließend wurden die optische Dichte (OD) ( Biochrom Novaspec 4049, LKB Instrument GmbH, Gräfelfing, Deutschland) bei einer Messwellenlänge von 600 nm und die Konzentration an gebildetem L-Alanin, L-Lysin und L-Valin mit einem Aminosäureanalysator der Firma Eppendorf-BioTronik (Hamburg, Deutschland) durch Ionenaustauschchromatographie und Nachsäulenreaktion mit Ninhydrindetektion bestimmt. In Tabelle 5 ist das Ergebnis des Versuchs dargestellt.

**Tabelle 5**

| Stamm | OD | L-Alanin g/l | L-Lysin g/l | L-Valin g/l |
|---|---|---|---|---|
| DSM5715 | 29,4 | Spuren | 1,6 | 0,1 |
| DSM5715/pEKExpgdh | 19,4 | 0,6 | 2,1 | 0,6 |

## Patentansprüche

1. Verfahren zur Herstellung von einer oder mehreren der L-Aminosäuren, ausgewählt aus der Gruppe der L-Threonin, L-Isoleucin, L-Valin und L-Tryptophan durch Fermentation coryneformer Bakterien,
**dadurch gekennzeichnet,**
**daß** man mit einem aus Mikroorganismen stammenden Glutamatdehydrogenase-Gen transformierte Bakterien einsetzt, in denen man die intrazelluläre Aktivität der Glutamatdehydrogenase erhöht, insbesondere die für das Glutamatdehydrogenase-Gen codierende Nukleotidsequenz überexprimiert.

2. Verfahren gemäß Anspruch 1,
**dadurch gekennzeichnet,**
**daß** man Bakterien einsetzt, in denen man zusätzlich die übrigen Gene des Stoffwechselweges der Bildung der gewünschten L-Aminosäuren verstärkt.

3. Verfahren gemäß Anspruch 1,
**dadurch gekennzeichnet,**
**daß** man Bakterien einsetzt, in denen die Stoffwechselwege zumindest teilweise ausgeschaltet sind, die die Bildung der gewünschten L-Aminosäure(n) verringern.

4. Verfahren gemäß einem oder mehreren der vorhergehenden Ansprüche,
**dadurch gekennzeichnet,**
**daß** man einen mit einem Plasmidvektor transformierten Stamm einsetzt und der Plasmidvektor die für die Glutamatdehydrogenase codierende Nucleotidsequenz trägt.

5. Verfahren gemäß Anspruch 6,
**dadurch gekennzeichnet,**
**daß** man mit dem Plasmidvektor pEK1,9gdh-1 hinterlegt in Corynebacterium glutamicum, unter der Nummer DSM 12614, transformierte Bakterium einsetzt.

6. Verfahren gemäß Anspruch 6,
**dadurch gekennzeichnet,**
**daß** man mit dem Plasmidvektor pEKExpgdh, hinterlegt in E.coli unter der Nummer DSM 12613, transformierte Bakterien einsetzt.

7. Verfahren zur Herstellung von einer oder mehreren der L-Aminosäuren, ausgewählt aus der Gruppe der L-Threonin, L-Isoleucin, L-Valin, und L-Tryptophan durch Fermentation coryneformer Bakterien gemäß Anspruch 1,
**dadurch gekennzeichnet,**
**daß** man folgende Schritte durchführt:
a) Fermentation von mit dem Glutamatdehydrogenase-Gen transformierten Bakterien, in denen man die intrazelluläre Aktivität der Glutamatdehydrogenase erhöht, insbesondere die für das Glutamatdehydrogenase-Gen codierende Nukleotidsequenz überexprimiert,
b) Anreicherung der gewünschten L-Aminosäure(n) im Medium oder in den Zellen der Bakterien und
c) Isolieren der L-Aminosäure(n).

## Claims

1. A process for the production of one or more of the L-amino acids selected from among the group L-threonine, L-isoleucine, L-valine and L-tryptophan by fermentation of coryneform bacteria,
**characterised in that**
bacteria transformed with a glutamate dehydrogenase gene originating from microorganisms are used, in which bacteria the intracellular activity of glutamate dehydrogenase is increased, in particular the nucleotide sequence coding for the glutamate dehydrogenase gene is overexpressed.

2. A process according to claim 1,
**characterised in that**
bacteria are used in which the remaining genes in the metabolic pathway for the formation of the desired L-amino acids are additionally amplified.

3. A process according to claim 1,
**characterised in that**
bacteria are used in which the metabolic pathways which reduce the formation of the desired L-amino acid(s) are at least partially switched off.

4. A process according to one of more of the preceding claims,
**characterised in that**
a strain transformed with a plasmid vector is used and the plasmid vector bears the nucleotide sequence coding for glutamate dehydrogenase.

5. A process according to claim 6,
**characterised in that**
a bacterium transformed with the plasmid vector pEK1.9gdh-1, deposited in *Corynebacterium glutamicum* under number DSM 12614, is used.

6. A process according to claim 6,
**characterised in that**
bacteria transformed with the plasmid vector pEKExpgdh, deposited in *E. coli* under the number DSM 12613, are used.

7. A process for the production of one or more of the L-amino acids selected from among the group L-threonine, L-isoleucine, L-valine and L-tryptophan by fermentation of coryneform bacteria according to claim 1,
**characterised in that**
the following steps are performed:
a) fermentation of the bacteria transformed with the glutamate dehydrogenase gene, in which bacteria the intracellular activity of glutamate dehydrogenase is increased, in particular the nucleotide sequence coding for the glutamate dehydrogenase gene is overexpressed,
b) accumulation of the desired L-amino acid(s) in the medium or in the cells of the bacteria and
c) isolation of the L-amino acid(s).

## Revendications

1. °)°Procédé de préparation d'un ou plusieurs acides aminés choisis dans le groupe constitué par la L-thréonine, la L-isoleucine,
la L-valine et le L-tryptophane par fermentation de bactéries corynéformes,
**caractérisé en ce qu'**
on utilise des bactéries transformées avec un gène de glutamate déshydrogénase provenant de microorganismes, en accroissant l'activité intracellulaire de la déshydrogénase, en particulier en surexprimant la séquence nucléotidique codant pour le gène de glutamate déshydrogénase.

2. °)°Procédé selon la revendication 1,
**caractérisé en ce qu'**
on utilise les bactéries dans lesquelles en outre on renforce les autres gènes des métabolismes de formation des acides L-aminés désirés.

3. Procédé selon la revendication 1,
**caractérisé en ce qu'**
on utilise des bactéries dans lesquelles sont au moins en partie mises hors service les voies métaboliques qui diminuent la formation du ou des acides aminés.

4. Procédé selon l'une ou plusieurs des revendications précédentes,
**caractérisé en ce qu'**
on utilise une souche transformée avec un vecteur plasmidique et en ce le vecteur plasmidique porte la séquence nucléotidique codant pour la glutamate déshydrogénase.

5. Procédé selon la revendication 1,
**caractérisé en ce qu'**
on utilise une bactérie transformée avec le vecteur plasmidique pEK1,9gdh-1 déposé dans Corynebacterium glutamicum dans DSM 12614.

6. Procédé selon la revendication 1,
**caractérisé en ce qu'**
on utilise des bactéries transformées avec le vecteur plasmidique pEKExpgdh, déposé dans E. coli sous le N° DSM 12613.

7. Procédé de préparation d'un ou plusieurs des acides L-aminés choisis dans le groupe constitué par la L-thréonine, la L-isoleucine, la L-valine et le L-tryptophane, par fermentation de bactéries corynéformes selon la revendication 1,
**caractérisé en ce qu'**
on réalise les étapes suivantes :
a) fermentation de bactéries transformées avec le gène de la glutamate déshydrogénase, dans lesquelles accroît l'activité intracellulaire de la glutamate déshydrogénase, en particulier on surexprime la séquence nucléotidique pour le gène de la glutamate déshydrogénase,
b) enrichissement du ou des acides L-aminés désirés dans le milieu ou dans les cellules de bactéries et
c) isolement du ou des acides L-aminés.
